# EUROPÄISCHE PATENTSCHRIFT

(11) **EP 0 985 659 B1**
(45) Veröffentlichungstag und Bekanntmachung des Hinweises auf die Patenterteilung: **29.10.2003**
(21) Anmeldenummer: 99115469.1
(22) Anmeldetag: 05.08.1999
(51) Int. Cl.: C07C 253/10, C07C 255/46

(54) **Verfahren zur Herstellung von 3-Cyano-3,5,5-trimethyl-cyclohexanon**
Process for the preparation of 3-cyano-3,5,5-trimethylcyclohexanone
Procédé pour la préparation de 3-cyano-3,5,5-triméthylcyclohexanone

(30) Priorität: 12.08.1998 DE 19836474
(43) Veröffentlichungstag der Anmeldung: 15.03.2000
(73) Patentinhaber: BASF AKTIENGESELLSCHAFT, 67056 Ludwigshafen (DE)
(72) Erfinder: Fischer, Jakob, Dr., 85414 Kirchdorf (DE); Siegel, Wolfgang, Dr., 67117 Limburgerhof (DE); Bomm,Volker, Dr., 67112 Mutterstadt (DE); Fischer, Martin, Dr., 67071 Ludwigshafen (DE); Mundinger, Klaus, Dr., 67117 Limburgerhof (DE)

(56) Entgegenhaltungen:
- EP-A- 0 671 384
- US-A- 5 183 915
- DATABASE WPI Section Ch, Week 199229 Derwent Publications Ltd., London, GB; Class E15, AN 1992-239923 XP002120049 & JP 04 164057 A (ASAHI GLASS CO LTD), 9. Juni 1992 (1992-06-09)

## Beschreibung

Die vorliegende Erfindung betrifft ein Verfahren zur Herstellung von 3-Cyano-3,5,5-trimethyl-cyclohexanon (Isophoronnitril, IPN) durch Umsetzung von Isophoron (IP) mit Cyanwasserstoff (HCN) in Gegenwart eines Katalysators bei Temperaturen von 80 bis 220 °C.

Isophoronnitril (IPN) ist ein Zwischenprodukt zur Herstellung von Isophorondiamin (IPDA), das als Epoxihärter oder als Monomer für Polyamine und Polyurethane verwendet wird.

Gemäß US-A-4,299,775 wird Isophoronnitril durch Umsetzung von Isophoron mit einer wässrigen Lösung eines Cyanids in Gegenwart eines Phasentransferkatalysators hergestellt.

Aus der US-A-5,011,968 ist ein Verfahren zur Herstellung von Isophoronnitril aus Isophoron und Cyanwasserstoff in Gegenwart von katalytischen Mengen eines quartären Ammoniumhydroxid-Katalysators vom Typ NR₄⁺OH⁻, wobei R Methyl, Ethyl oder Butyl bedeutet, bekannt.

JP-A-61 033 157 (Chem. Abstr. 105, 42383u) beschreibt die Umsetzung von Isophoron mit HCN zu Isophoronnitril in Gegenwart eines quartären Ammoniumhydroxids, wie z.B Tetrabutylammoniumhydroxid, oder quartären Phosphoniumhydroxids als Katalysator.

Aus der EP-A-502 707 (Seite 3, Zeilen 12-17) ist bekannt, daß bei Verwendung von quartären Ammonium- oder quartären Phosphoniumhydroxiden als Katalysatoren für die IPN-Synthese aus HCN und Isophoron, diese aufgrund ihrer starken Basizität in ungewünschter Weise zu HCN-Polymerisation und zu Polymerisation des Isophoronnitrils führen.

In der EP-A-502 707 und der äquivalenten US-A-5,179,221 wird zur Synthese von IPN aus Isophoron und HCN als Katalysator die Verwendung von Tetraalkylammoniumhalogeniden oder quartären Phosphoniumhalogeniden unter Zusatz einer basischen Komponente beschrieben.

Ein allen oben genannten Verfahren zur IPN-Herstellung gemeinsamer Nachteil ist, daß die Umsetzungen bevorzugt in Gegenwart von Wasser durchgeführt werden. Bei der destillativen Aufarbeitung der Reaktionsauträge kommt es deshalb zu Verlusten an stets im Überschuß eingesetztem Isophoron, da Isophoron bekanntermaßen ein Azeotrop mit Wasser bildet. Weiterhin nachteilig ist die notwendige Entsorgung des verunreinigten Wassers.

Aus der US-A-5,183,915 ist ein Verfahren zur Herstellung von IPN aus Isophoron und Cyanwasserstoff in Gegenwart von quartären Ammoniumcyanid- oder quartären Phosphoniumcyanid-Katalysatoren bekannt. Diese Katalysatoren sind jedoch teuer und die IPN-Ausbeuten sind verbesserungsbedürftig.

Die DE-A-44 07 487 beschreibt ein Verfahren zur Herstellung von IPN durch Umsetzung von Isophoron mit HCN in Gegenwart von Tetraalkylammoniumhydrogencarbonaten oder Tetraalkylammoniumalkylcarbonaten als Katalysator.

Wie allgemein bekannt ist, sind Tetraalkylammoniumionen in Gegenwart einer Base, wie z.B. einem basischen Anion, thermisch nicht stabil und werden zu einem Trialkylamin zersetzt (Hofmann-Eliminierung; siehe z.B.: R.T. Morrison und R.N. Boyd, Organic Chemistry, 6^{th} Ed., 1992, Seite 854 unten bis Seite 855 oben). Ist diese Base beispielsweise ein Hydroxid-Ion, so tritt die Zersetzung der Tetraalkylammoniumionen ab ca. 125 °C ein.

Der vorliegenden Erfindung lag daher die Aufgabe zugrunde, den zuvor genannten Nachteilen abzuhelfen und ein alternatives- Verfahren zur Herstellung von IPN aus Isophoron und HCN aufzufinden, das das Produkt in hohen Raum-Zeit-Ausbeuten, bezogen auf die Edukte und auf den Katalysator, liefert.

Demgemäß wurde ein neues und verbessertes Verfahren zur Herstellung von 3-Cyano-3,5,5-trimethyl-cyclohexanon (Isophoronnitril, IPN) durch Umsetzung von Isophoron mit Cyanwasserstoff in Gegenwart eines Katalysators bei Temperaturen von 80 bis 220 °C gefunden, welches dadurch gekennzeichnet ist, daß man die Umsetzung in Gegenwart des Betains 1,3-Dimethyl-imidazolium-4-carboxylat durchführt.

Die katalytisch aktive Betain-Verbindung weist die in Formel I dargestellte Struktur auf:

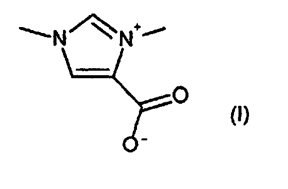

Die Verbindung I ist als Naturstoff unter dem Namen Norzooanemonin bekannt, dessen Isolierung und Charakterisierung in Tetrahedron Lett. 38, 3883-4 (1997) und in Tetrahedron 29, 3135-6 (1973) beschrieben wurde.

In Tetrahedron 29, 3135-6 (1973) wird auch die Synthese von 1,3-Dimethyl-imidazolium-4-carboxylat (I) durch Umsetzung von kommerziell erhältlicher Imidazol-4-carbonsäure mit Dimethylsulfat unter pH-Kontrolle beschrieben. Das zunächst durch die Umsetzung von Imidazol-4-carbonsäure mit Dimethylsulfat erhaltene Betain I, das noch mit Natriummethylsulfat verunreinigt ist, kann direkt, ohne einen weiteren Aufarbeitungsschritt, als Katalysator im erfindungsgemäßen Verfahren zur Herstellung von IPN aus Isophoron und HCN eingesetzt werden.
Das Betain I wird jedoch im allgemeinen zunächst, wie in loc. cit. zur Synthese von I beschrieben, gereinigt und anschließend als Katalysator im erfindungsgemäßen Verfahren eingesetzt.

Bevorzugt wird das 1,3-Dimethyl-imidazolium-4-carboxylat (I) durch Umsetzung von 1-Methyl-imidazol mit Dimethylcarbonat gemäß der zeitgleichen deutschen Anmeldung Nr. 19836477.6 hergestellt und als Katalysator im erfindungsgemäßen Verfahren eingesetzt.

Das erfindungsgemäße Verfahren zur Herstellung von IPN aus Isophoron und HCN läßt sich wie folgt durchführen:

Man kann beispielsweise
a) Isophoron mit dem Katalysator der Formel I vorlegen und Cyanwasserstoff in einem inerten Lösungsmittel oder in Isophoron zugeben oder
b) Isophoron mit Cyanwasserstoff vorlegen und den Katalysator der Formel I in einem inerten Lösungsmittel oder Isophoron zugeben oder
c) Isophoron vorlegen und Cyanwasserstoff und den Katalysator der Formel I in einem inerten Lösungsmittel oder Isophoron zugeben.

Die Variante a) ist hierbei bevorzugt.

Das Verfahren kann bei Reaktionstemperaturen von 80 bis 220 °C, bevorzugt 100 bis 180 °C, besonders bevorzugt 120 bis 170 °C, durchgeführt werden.

Der Reaktionsdruck (absolut gemessen) beträgt in der Regel 0,05 bis 2 MPa, bevorzugt 0,09 bis 1 MPa, besonders bevorzugt Atmosphärendruck (Normaldruck).

Der Katalysator der Formel I wird in der Regel in Mengen von 0,005 bis 5 Mol-%, bevorzugt 0,01 bis 2 Mol-%, besonders bevorzugt 0,05 bis 1 Mol-%, bezogen auf den eingesetzten Cyanwasserstoff verwendet.

Im erfindungsgemäßen Verfahren wird in der Regel das Isophoron im molaren Überschuß bezogen auf den Cyanwasserstoff eingesetzt. Das molare Verhältnis der beiden Einsatzstoffe Isophoron und Cyanwasserstoff kann IP : HCN = 2 bis 10 : 1, bevorzugt 2 bis 5 : 1, besonders bevorzugt 2 bis 3 : 1, betragen.

Das nach der Reaktion unumgesetzte Isophoron kann durch Destillation zurückgewonnen werden.

Mit dem erfindungsgemäß verwendeten Katalysator sind besonders kurze Verweilzeiten des Reaktionsgemisches im Reaktor bei guten Ausbeuten, Selektivitäten und hohen Raum-Zeit-Ausbeuten erzielbar. Je nach den gewählten Reaktionsbedingungen betragen die Verweilzeiten im allgemeinen 20 Minuten bis wenige Stunden.

Weiterhin wird die als Nebenreaktion bekannte Oligomerisation bzw. Polymerisation der beiden Einsatzstoffe Isophoron und Cyanwasserstoff nahezu vollständig vermieden und der nach der destillativen Aufarbeitung des Reaktionsaustrags zu entsorgende Destillationsrückstand dadurch minimiert.

Als Reaktionsgefäße bzw. Reaktoren eignen sich beispielsweise Rührreaktoren, Rohrreaktoren, Rührbehälterkaskaden oder Mischkreise.

Das erfindungsgemäße Verfahren kann diskontinuierlich oder bevorzugt kontinuierlich durchgeführt werden.

Beispielsweise kann eine kontinuierliche Verfahrensdurchführung derart erfolgen, daß der Katalysator der Formel I, gegebenenfalls in einem inerten Lösungsmittel oder in Isophoron gelöst, kontinuierlich in eine Apparatur gespeist wird, in der kontinuierlich Isophoron mit Cyanwasserstoff unter Normaldruck oder unter erhöhtem Druck (0,09 bis 1 MPa, absolut gemessen) umgesetzt wird.

Die Umsetzung kann in Gegenwart oder Abwesenheit von inerten Lösungsmitteln vorgenommen werden.

Als inerte Lösungsmittel für die Umsetzung eignen sich Wasser und C₁- bis C₂₀-Alkanole, bevorzugt C₁- bis C₈-Alkanole, besonders bevorzugt C₁- bis C₄-Alkanole, wie Methanol, Ethanol, n-Propanol, iso-Propanol, n-Butanol, iso-Butanol und tert.-Butanol, aliphatische Kohlenwasserstoffe mit 5 bis 30 C-Atomen, bevorzugt mit 5 bis 20 C-Atomen, besonders bevorzugt mit 5 bis 10 C-Atomen, wie n-Pentan, Pentanisomerengemische, n-Hexan, Hexanisomerengemische, n-Heptan, Heptanisomerengemische, n-Octan, Octanisomerengemische, cycloaliphatische Kohlenwasserstoffe mit 5 bis 20 C-Atomen, bevorzugt mit 5 bis 12 C-Atomen, besonders bevorzugt mit 5 bis 8 C-Atomen, wie Cyclopentan, Cyclohexan, Cycloheptan und Cyclooctan, Amide, wie Dimethylformamid, Dimethylacetamid, N-Methylpyrrolidon, Harnstoffe, wie N,N'-Dimethylethylenharnstoff, N,N'-Dimethylpropylenharnstoff, N,N,N',N'-Tetra-n-butylharnstoff, oder Carbonate, wie Ethylencarbonat und Propylencarbonat.

Besonders bevorzugt verwendet man Isophoron im molaren Überschuß, bezogen auf HCN, und setzt kein externes Lösungsmittel zu.

Zur Neutralisation des Reaktionsaustrages können Säuren, z.B. anorganische Säuren, wie Phosphorsäure und Schwefelsäure, oder organische Säuren, beispielsweise Sulfonsäuren, wie Methansulfonsäure, Toluolsulfonsäure, Carbonsäuren, wie Ameisensäure, Essigsäure, Propionsäure, Malonsäure, 2-Ethyl-hexansäure und Adipinsäure, verwendet werden.

### Beispiele

In den nachfolgenden Beispielen zur Herstellung von IPN erfolgte die Ermittlung der HCN-Konzentration in den Reaktionsmischungen durch salpetersaure Titration nach Volhard (Vorlage von 0,1 molarer AgNO₃-Lösung, Zugabe der zu analysierenden Probe und Rücktitration mit 0,1 molarer KSCN-Lösung).

### Beispiel 1

### Herstellung von Isophoronnitril (IPN)

Es wurden 622 g (4,5 mol) Isophoron und 1,8 g (12,9 mmol) 1,3-Dimethyl-imidazolium-4-carboxylat vorgelegt und innerhalb von ca. 60 Min. bei 150 °C eine Mischung aus 207,3 g (1,5 mol) Isophoron und 81,0 g (3 mol) Cyanwasserstoff zugegeben. Anschließend wurde bei 150 °C ca. 60 Min. nachgerührt. Nach dem Erkalten wurde die HCN-Konzentration mittels Titration gemessen. Es konnte keine freie Blausäure nachgewiesen werden (HCN-Umsatz > 99,9 %). Nach Zugabe von 2,0 g 85%iger H₃PO₄ wurde der Reaktionsaustrag bei 0,1 hPa destilliert. Man erhielt 486 g 3-Cyano-3,5,5-trimethyl-cyclohexanon (98,1 % bezogen auf eingesetzte HCN). Die Menge an Destillationsrückstand betrug lediglich 3 Gew.-%, bezogen auf eingesetztes Isophoron.

### Beispiel 2

### Herstellung von Isophoronnitril

Es wurden 622 g (4,5 mol) Isophoron und 3,61 g (25,8 mmol) 1,3-Dimethyl-imidazolium-4-carboxylat vorgelegt und innerhalb von ca. 120 Min. bei 120 °C eine Mischung aus 207,3 g (1,5 mol) Isophoron und 81,0 g (3 mol) Cyanwasserstoff zugegeben. Anschließend wurde bei 120 °C ca. 120 Min. nachgerührt. Nach dem Erkalten wurde die HCN-Konzentration mittels Titration gemessen. Es konnte keine freie Blausäure mehr nachgewiesen werden (HCN-Umsatz > 99,9 %). Nach Zugabe von 2,0 g 85%iger H₃PO₄ wurde der Reaktionsaustrag bei 0,1 hPa destilliert. Man erhielt 473 g 3-Cyano-3,5,5-trimethylcyclohexanon (95,4 % bezogen auf eingesetzte HCN).

### Beispiel 3

### Herstellung von Isophoronnitril

Es wurden 622 g (4,5 mol) Isophoron und 3,61 g (25,8 mmol) 1,3-Dimethyl-imidazolium-4-carboxylat vorgelegt und innerhalb von ca. 60 Min. bei 120 °C eine Mischung aus 207,3 g (1,5 mol) Isophoron und 81,0 g (3 mol) Cyanwasserstoff zugegeben. Anschließend wurde bei 120 °C ca. 60 Min. nachgerührt. Nach dem Erkalten wurde die HCN-Konzentration mittels Titration gemessen. Es konnte keine freie Blausäure mehr nachgewiesen werden (HCN-Umsatz > 99,9 %). Nach Zugabe von 2,0 g 85%iger H₃PO₄ wurde der Reaktionsaustrag bei 0,1 hPa destilliert. Man erhielt 484 g 3-Cyano-3,5,5-trimethylcyclohexanon (97,7 % bezogen auf eingesetzte HCN).

### Vergleichsbeispiel 1 zur Herstellung von IPN

Zu 415 g (3 mol) Isophoron und 12 g (0,077 mol) Tetraethylammoniumcyanid wurden bei 108 °C innerhalb von 60 Min. 81 g (3 mol) Cyanwasserstoff zugetropft. Nach einer Nachreaktionszeit von 30 Min. betrug die HCN-Konzentration 0,16 %, d.h. der HCN-Umsatz betrug 99 %. Der Reaktionsaustrag wurde mit 9 g H₃PO₄ neutralisiert und destilliert. Man erhielt 15 g Isophoron und 443,6 g 3-Cyano-3,5,5-trimethyl-cyclohexanon. Das entsprach einer Ausbeute von 89,6 % bezogen auf eingesetzte HCN und 93 % bezogen auf umgesetztes Isophoron. Der Destillationsrückstand betrug 28,2 g (= 6,8 Gew.-% bezogen auf eingesetztes Isophoron).

### Vergleichsbeispiel 2a zur Herstellung von IPN

Es wurden 622 g (4,5 mol) Isophoron und 4,47 g (30 mmol) Tetramethylammoniummethylcarbonat vorgelegt und innerhalb von ca. 60 Min. bei 120 °C 288,3 g einer Mischung aus 1,5 mol Isophoron und 3 mol Cyanwasserstoff zugegeben. Nach Beendigung der Dosierung betrug die HCN-Konzentration in der Reaktionsmischung 20 ppm, d.h. der HCN-Umsatz betrug > 99,9 %. Anschließend wurden 3,5 g 85%ige H₃PO₄ zugegeben und der Reaktionsaustrag bei 0,1 hPa destilliert. Man erhielt 426,6 g Isophoron und 476,8 g 3-Cyano-3,5,5-trimethyl-cyclohexanon (96,2 % bezogen auf eingesetzte HCN).

### Vergleichsbeispiel 2b zur Herstellung von IPN

Es wurden 622 g (4,5 mol) Isophoron und 4,47 g (30 mmol) Tetramethylammoniummethylcarbonat vorgelegt und innerhalb von ca. 120 Min. bei 140 °C 288,3 g einer Mischung aus 1,5 mol Isophoron und 3 mol Cyanwasserstoff zugegeben. Noch vor Beendigung der Dosierung fiel die Temperatur stark ab und man erhielt Rückfluß (unumgesetzte HCN). Nach Beendigung der Dosierung wurde 120 Min. bei Rückfluß nachgerührt. Durch Titration wurde der HCN-Umsatz zu lediglich 92 % gemessen. Der Reaktionsansatz wurde nicht weiter aufgearbeitet.

## Patentansprüche

1. Verfahren zur Herstellung von 3-Cyano-3,5,5-trimethyl-cyclohexanon durch Umsetzung von Isophoron mit Cyanwasserstoff in Gegenwart eines Katalysators bei Temperaturen von 80 bis 220°C, **dadurch gekennzeichnet, daß** man die Umsetzung in Gegenwart des Betains 1,3-Dimethyl-imidazolium-4-carboxylat durchführt.

2. Verfahren nach Anspruch 1, **dadurch gekennzeichnet, daß** man die Umsetzung in Gegenwart von 0,005 bis 5 Mol-% des Betains bezogen auf Cyanwasserstoff durchführt.

3. Verfahren nach den Ansprüchen 1 und 2, **dadurch gekennzeichnet, daß** man die Umsetzung bei Temperaturen von 100 bis 180°C durchführt.

4. Verfahren nach den Ansprüchen 1 bis 3, **dadurch gekennzeichnet, daß** man die Umsetzung bei Absolutdrücken von 0,09 bis 1 MPa durchführt.

5. Verfahren nach den Ansprüchen 1 bis 4, **dadurch gekennzeichnet, daß** man die Umsetzung bei Atmosphärendruck durchführt.

## Claims

1. A process for preparing 3-cyano-3,5,5-trimethylcyclohexanone by reacting isophorone with hydrogen cyanide at from 80 to 220°C in the presence of a catalyst, wherein the reaction is carried out in the presence of the betaine 1,3-dimethylimidazolium-4-carboxylate.

2. A process as claimed in claim 1, wherein the reaction is carried out in the presence of from 0.005 to 5 mol% of the betain, based on hydrogen cyanide.

3. A process as claimed in claim 1 or 2, wherein the reaction is carried out at from 100 to 180°C.

4. A process as claimed in any of claims 1 to 3, wherein the reaction is carried out at absolute pressures of from 0.09 to 1 MPa.

5. A process as claimed in any of claims 1 to 4, wherein the reaction is carried out at atmospheric pressure.

## Revendications

1. Procédé pour la préparation de la 3-cyano-3,5,5-triméthyl-cyclohexanone au moyen de la réaction de l'isophorone avec de l'acide cyanhydrique en présence d'un catalyseur, à une température allant de 80°C à 220°C, **caractérisé en ce que** l'on réalise la réaction en présence de la bétaïne 1,3-diméthyl-imidazolium-4-carboxylate.

2. Procédé selon la revendication 1, **caractérisé en ce que** l'on réalise la réaction en présence de 0,005% en moles à 5% en moles de bétaïne par rapport à l'acide cyanhydrique.

3. Procédé selon les revendications 1 et 2, **caractérisé en ce que** l'on réalise la réaction à une température allant de 100°C à 180°C.

4. Procédé selon les revendications 1 à 3, **caractérisé en ce que** l'on réalise la réaction sous une pression absolue allant de 0,09 MPa à 1 MPa.

5. Procédé selon les revendications 1 à 4, **caractérisé en ce que** l'on réalise la réaction sous la pression atmosphérique.
